# EUROPEAN PATENT SPECIFICATION

(11) **EP 3 332 657 B1**
(45) Date of publication and mention of the grant of the patent: **05.08.2020**
(21) Application number: 17205555.0
(22) Date of filing: 05.12.2017
(51) Int. Cl.: A24F 47/00, A61M 15/06, A61M 11/04, A61M 15/00

(54) **HEATING ELEMENT, VAPORIZATION DEVICE AND METHOD FOR VAPORIZING FLUID EJECTED BY EJECTION HEAD**
HEIZELEMENT, VERDAMPFUNGSVORRICHTUNG UND VERFAHREN ZUR VERDAMPFUNG EINER FLÜSSIGKEIT DURCH AUSSTOSSKOPF
ÉLÉMENT CHAUFFANT, DISPOSITIF DE VAPORISATION ET PROCÉDÉ DE VAPORISATION DE FLUIDE ÉJECTÉ PAR UNE TÊTE D'ÉJECTION

(30) Priority: 06.12.2016 US 201615369961
(43) Date of publication of application: 13.06.2018
(73) Proprietor: Funai Electric Co., Ltd., Daito, Osaka, 574-0013 (JP)
(72) Inventor: BELL, Byron V., Lexington, KY Kentucky 40511 (US)
(74) Representative: 2K Patentanwälte Blasberg Kewitz & Reichel

(56) References cited:
- EP-A1- 0 845 220
- WO-A1-2016/108694
- US-A1- 2003 007 035
- US-A1- 2011 226 236
- US-A1- 2013 220 314
- US-A1- 2015 114 409
- US-A1- 2016 309 785

## Description

### TECHNICAL FIELD

The disclosure relates to a heating element, a vaporization device, and a method for vaporizing a fluid ejected by an ejection head, and in particular to a heating element, a vaporization device, and a method for vaporizing a fluid ejected by an ejection head with improved evaporation efficiency.

### BACKGROUND

When jetting a fluid onto a heated surface of a vaporizing heater, it is highly desirable for 100% of the fluid to be vaporized, so that liquid is not discharged from the vaporizing heater of a vaporization device. The problem lies in that the vaporizing heater must be small enough to heat up extremely quickly, but yet has enough surface area to catch all fluid and fluid droplets that are being ejected onto a heating element. A typical metal foil heating element has a smooth surface with minimal liquid/heater interface which is due to a low surface roughness of a heating element surface. Accordingly, some of the fluid droplets impinging on the surface of the heating element will be scattered or fluid droplets will be ejected from the heating element if a significant layer of fluid already exists on the surface of the heating element, when new droplets arrive. Thus, instead of only vapor being discharged from the vaporization device, liquid droplets may be entrained in the vapor and discharged from the vaporization device. In some applications, the discharge of liquid is undesirable. Also, unvaporized fluid may build up inside the vaporization device and thus degrade the operation of the vaporization device. US2011/226236 A1 relates to an inhaler component for producing a steam/air mixture or/and condensation aerosol in an intermittent and inhalation- or pull-synchronous manner. Said inhaler component includes the following elements: a housing (3); a chamber (21) arranged in said housing (3); an air inlet opening (26) for the supply of air from the surroundings to the chamber (21); an electrical heating element for evaporating a portion of a liquid material (16), the steam produced mixing in the chamber (21) with the air supplied through the air inlet opening (26), thereby producing the steam/air mixture or/and condensation aerosol; and a wick having a capillary structure, which wick forms a composite structure (22) with the heating element and automatically supplies the heating element with fresh liquid material (16) after evaporation. In order to provide the high specific evaporative capacity required for an intermittent, inhalation- or pull-synchronous operation of the inhaler component (2) while guaranteeing a high efficiency of the evaporator, the composite structure (22) is a flat structure and at least one heated section of the composite structure (22) is arranged in the chamber (21) in a contact-free manner, and the capillary structure of the wick is substantially exposed in said section on at least one side (24) of the flat composite structure. WO2016108694 A1 relates to a personal electronic delivery system and a method for delivering a delivery fluid to a person. The system according to the invention comprises: - a housing having a first end with an inlet (12) and a second end with an outlet (38); - a fluid path substantially extending between the inlet and the outlet; - a buffer (30) for holding a delivery fluid, and connecting means configured to transfer delivery fluid to the fluid path; and a heater (32) that is provided in, at or close to the fluid path configured for heating the delivery fluid such that at least a part of the delivery fluid atomizes and/or vaporizes in the fluid path, and an energy source (18) configured for providing energy to the heater. EP0845220 (A1) discloses a flavor generation article (10) having a casing (12) constituted by first and second portions (12a, 12b) that are detachably connected to each other. A gas flow path (26) is formed in the casing first portion (12a) to extend from an air intake port (24) to reach a suction port (22). The first portion (12a) incorporates a material container (32) of a liquid material (36) containing a flavor substance. A discharge port (35) of the material container (32) is arranged in the gas flow path (26), and a ceramic heater (42) is disposed to oppose it. The liquid material (36) is supplied from the discharge port (35) onto the ceramic heater (42) and is heated, so that it is gasified in the gas flow path (26). The casing second portion (12b) incorporates a control circuit (72) and a power supply (62). US2013220314 A1 discloses an apparatus and method utilize a porous vaporization element with a dispersion mechanism to disperse a liquid agent onto the porous vaporization element. The porous vaporization element extends across a majority of a cross-sectional area of the flow passage. In one implementation, the liquid agent is sprayed onto the porous vaporization element, US2016309785 A1 discloses a power supply section for an e-vapor device includes a power supply section housing including a power supply portion and a heater assembly portion. The power supply portion includes a power supply, and the heater assembly portion includes a heating element and a support. The power supply is configured to selectively supply power to the heating element. The heating element includes a planar portion and first and second lead portions. The planar portion includes at least one filament. The first and second lead portions extend away from the planar portion. The support may support the heating element in the power supply section housing. The support includes a first slot and a second slot, and the first lead portion extends through the first slot, and the second lead portion extends through the second slot. US2015114409 A1 relates to aerosol delivery devices. The aerosol delivery devices include mechanisms configured to deliver an aerosol precursor composition from a reservoir to an atomizer including a vaporization heating element to produce a vapor. For example, a bubble jet head may be configured to dispense the aerosol precursor composition to the atomizer. The bubble jet head may be fixedly coupled to the atomizer. The bubble jet head may include a precursor inlet, an ejection heating element, and a precursor nozzle. The atomizer may include a vaporization heating element.

US2003/007035 A1 provides passivation layer for a thermal ink jet printhead. The material of the passivation layer can be a Co-based alloy with 25-30 wt % Cr or an Fe-based alloy with <=10 wt. % Co, <=20 wt. % Cr, <=10 wt. % Mn. The passivation layer is amorphous and the surface is substantially atomically smooth or has a controlled surface roughness. The passivation layer displays resistance to cavitation and chemical corrosion and is conformally disposed over a resistor by sputtering or other physical vapor deposition techniques.

### SUMMARY

In order to avoid the discharge of liquid droplets from a vaporization device, a stream of fluid ejected onto a surface of a heating element must be efficiently captured by the heating element, spread out over the surface of the heating element, and completely vaporized at approximately the same rate as the fluid arrives on the surface of the heating element, in order to avoid liquid accumulation on the surface of the heating element.

In view of the foregoing, embodiments of the disclosure provide a heating element for a vaporization device according to claim 1.

In one embodiment, the heating element is configured to have a rectangular shape and has the effective surface area for fluid vaporization defined by an equation ESA > L x W, wherein ESA is the effective surface area, L is a length of the heating element, and W is a width of the heating element.

In one embodiment, the porous layer has a grit blasted surface texture.

In one embodiment, the porous layer has a thickness ranging from about 0.5 mm to about 3.0 mm.

In one embodiment, the substrate, the conductive layer, the protective layer and the porous layer have a combined thickness ranging from about 4.0 mm to about 1.0 cm.

In one embodiment, the porous layer has a porosity ranging from about 50% to about 95%.

In one embodiment, the conductive layer is a screen printed conductive layer configured to be deposited onto a ceramic substrate.

In one embodiment, the porous layer is a laser etched ceramic layer.

In one embodiment, the porous layer is a coarse grit deposited ceramic layer that is sintered.

Another embodiment of the disclosure provides a vaporization device that includes: a housing body; a mouthpiece, configured to be attached to the housing body; and a heating element according to any one of the above embodiments, disposed adjacent to the mouthpiece for vaporizing fluid ejected from an ejection head.

A further embodiment of the disclosure provides a method for vaporizing a fluid ejected by an ejection head according to claim 11.

In one embodiment, the method further includes: absorbing the fluid by the porous layer of the heating element, wherein the porosity of the heating element is defined by an equation ESA > L x W, wherein ESA is the effective surface area of the heating element, L is a length of the heating element, W is a width of the heating element, and the heating element is configured to have a rectangular shape and the heating element is exposed to the fluid.

In one embodiment, the porous layer is deposited as a coarse glass frit that is sintered onto the protective layer of the heating element.

In the disclosure, an improved heating elements and methods for improving the vaporization efficiency of heating elements for vaporization devices can be obtained.

### BRIEF DESCRIPTION OF THE DRAWINGS

Other features and advantages of disclosed embodiments may be evident by reference to the following detailed description, drawings and claims wherein:
FIG. 1 is a cross-sectional view, not to scale, of a vaporization device according to an embodiment of the disclosure.
FIG. 2 is a close-up view, not to scale, of a portion of the vaporization device of FIG. 1.
FIG. 3 is a cross-sectional view, not to scale, of a heating element according to an embodiment of the disclosure.
FIG. 4 is a cross-sectional view, not to scale, of a heating element according to another embodiment of the disclosure.
FIG. 5-FIG. 7 are schematic views, not to scale, of a process for making a heating element according to an embodiment of the disclosure.
FIG. 8 is a cross-sectional view, not to scale, of a heating element having fluid absorbed into an upper porous surface according to an embodiment of the disclosure.

### DETAILED DESCRIPTION OF EMBODIMENTS

One of the applications of a fluidic ejection device is to jet a solution onto to another device where a secondary function may be performed. A common secondary function is to vaporize a solution using a heater such that the contents of the solution can be vaporized so as to deliver the solution as a gaseous substance. Applications of such technology include, but are not limited to, metering and vaporization device for electronic cigarettes, vapor therapy, gaseous pharmaceutical delivery, vapor phase reactions for micro-labs, and the like. A problem associated with such devices is efficient vaporization of the fluid. This disclosure describes improved heating elements and methods for improving the vaporization efficiency of heating elements for vaporization devices.

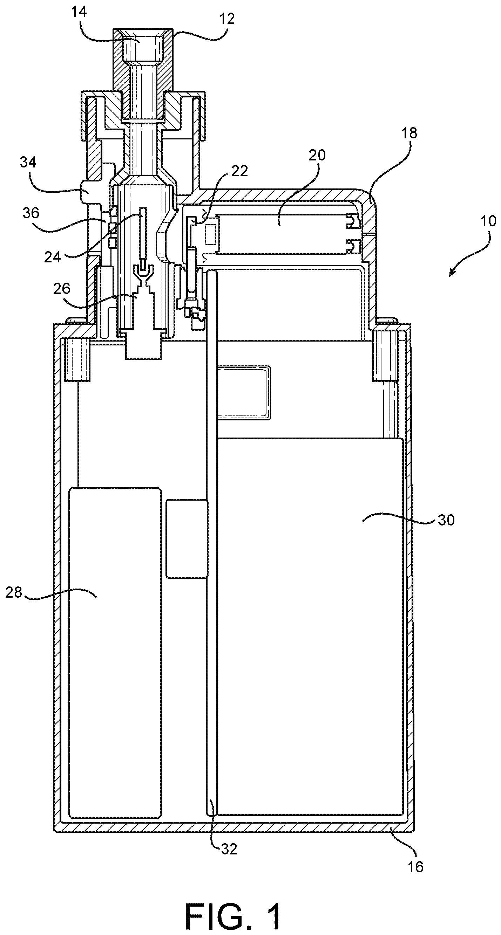

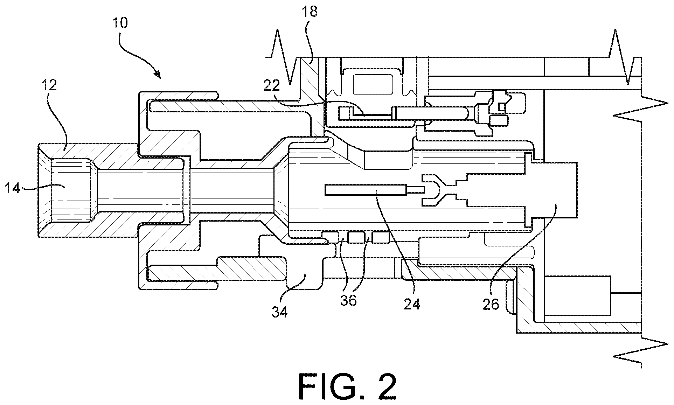

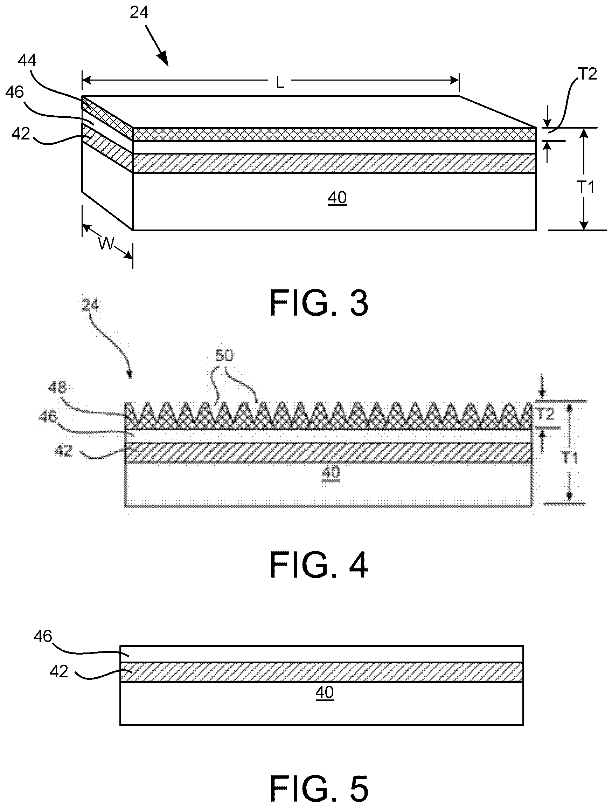

The disclosure is directed to a vaporization device 10 as shown in FIG. 1 and FIG. 2, and heating elements therefor as shown in FIG. 3-FIG. 8. The vaporization device 10 may be used for a wide variety of applications, wherein a liquid is ejected onto a heating element to provide a vapor stream as described in more detail below. The vaporization device 10 is typically a handheld device such as an electronic cigarette that has a mouthpiece 12 for inhaling vapors generated by the vaporization device 10. The mouthpiece 12 includes a conduit 14 for flow of vapors out of the vaporization device 10. Main components of the vaporization device 10 include a housing body 16, a removable cartridge cover 18, a removable fluid supply cartridge 20, an ejection head 22 associated with the fluid supply cartridge 20, and a heating element 24 and a holder 26 therefor for vaporizing fluid ejected from the ejection head 22. Other components associated with the vaporization device 10 include a rechargeable power supply 28, a main circuit board 30, and a vaporization driver card 32. An enlarged portion of the vaporization device 10 is shown in FIG. 2.

The mouthpiece 12, as well as the housing body 16 of the vaporization device 10 may be made from a wide variety of materials including plastics, metals, glass, ceramic and the like provided the materials are compatible with the fluids to be ejected and vaporized by the vaporization device 10. A particularly suitable material may be selected from polyvinyl chloride, high density polyethylene, polycarbonate, stainless steel, surgical steel, nickel-plated steel, and the like. All parts, including the mouthpiece 12, and the housing body 16 that come in contact with fluids and vapors may be made of plastic. The conduit 14 may be made of metal such as stainless steel or other material that is resistant to heat and vapors generated by the vaporization device 10.

As shown in FIG. 1, the housing body 16 may include the main circuit board 30 and the vaporization driver card 32 for providing the logic circuitry for the heating element 24 (described in more detail below) and the ejection head 22. The rechargeable power supply 28 may also be housed in the housing body 16. In another embodiment, a removable, non-rechargeable battery may be housed in the housing body 16. Electrical contacts, such as a Universal-Serial-Bus (USB, not shown) may be used to recharge the rechargeable power supply 28 and to change program setting for the ejection head 22 and the heating element 24. The microfluidic ejection head 22 is in fluid flow communication with the fluid supply cartridge 20 that provides fluid to be ejected by the ejection head 22.

An inlet air flow control device may be included to provide backpressure control on the ejection head 22. The inlet air flow control device may include a damper slide 34 and an air inlet hole 36 that allows air to be drawn into the conduit 14 adjacent the heating element 24 and the ejection head 22, so that excessive negative pressure on the ejection head 22 can be avoided.

An important component of the vaporization device 10 is the heating element 24 as shown in FIG. 3. The heating element 24 is typically made of a high temperature solid ceramic base (substrate) 40 having a resistive or conductive layer 42 printed thereon, deposited thereon, or otherwise imbedded in the ceramic base 40. A material of the resistive or conductive layer 42 may be selected from a wide variety of materials typically used for heating elements including, but not limited to, silver and/or carbon screen printed materials, tungsten, molybdenum, molybdenum-manganese, and the like.

As set forth above, it is desirable to vaporize substantially all fluid ejected from the ejection head 22, so that only vapors are discharged through the conduit 14 of the mouthpiece 12. Accordingly, the heating element 24 desirably contains a fluid absorbing or capturing layer 44 that is formed on a protective layer 46. The protective layer 46 is positioned between the fluid absorbing or capturing layer 44 and the resistive or conductive layer 42, and may be made of the same material as the ceramic base 40 or any other suitable, high temperature material that is substantially non-porous. Other suitable materials for the protective layer 46 include, but are not limited to alumina, aluminum nitride, silica or silicon nitride.

The overall thickness T1 of the heating element 24 may range from about 4.0 millimeters to about 1.0 centimeter. In other words, in an embodiment, a substrate 40, a resistive or conductive layer 42, a protective layer 46 and a fluid absorbing or capturing layer 44 (i.e., porous layer 44, see below for details) have a combined thickness ranging from about 4.0 mm to about 1.0 cm. The thickness T2 of the fluid absorbing or capturing layer 44 may range from about 0.5 millimeters to about 3 millimeters in thickness, such as from about 1 millimeter to about 2 millimeter in thickness. The thicknesses of the resistive or conductive layer 42 and the protective layer 46 are not critical to the embodiments of the disclosure.

In one embodiment, as shown in FIG. 3, the fluid absorbing or capturing layer 44 is a porous layer having a porosity of at least about 50% that is deposited onto the protective layer 46. In another embodiment, the porosity of the fluid absorbing or capturing layer 44 may range from about 60 % to about 85%. Having a porosity of at least about 50 % means that the fluid absorbing or capturing layer 44 is porous or has indentations or cavities that provide at least 50% void space volume relative to the entire volume of fluid absorbing or capturing layer 44. The porosity range is based on engineering judgement as the practical limits for a porous layer. The mass of the fluid absorbing or capturing layer 44 is as small as possible to optimize warm up speed and minimize power consumption for heating the fluid absorbing or capturing layer 44. Low mass requires a high porosity with 95% chosen as a realistic upper limit. Above 95% porosity the structure would be too weak and difficult to fabricate. A 50% porosity is chosen as the minimum porosity for the fluid absorbing or capturing layer 44. Below 50% porosity wicking properties of the fluid absorbing or capturing layer 44 will suffer due to closed off/inaccessible pores in the structure.

For a rectangular heating element having a width W, a length L and a thickness T1 as described above, the heating element 24 is further defined as having an effective surface area (ESA) for fluid vaporization that is defined by the equation ESA > L x W. For a circular heating element, the effective surface area (ESA) of the heating element is defined by the equation ESA > Π x R² wherein R is a radius of the heating element that is exposed to a vaporizing fluid. The heating element 24 is not limited to a rectangular or circular shape as any shape including triangular, complex shapes, and the like may be used. Accordingly, the ESA of the heating element 24 is greater than the nominal dimensions of the protective layer 46.

In another embodiment, as shown in FIG. 4, a grit blasted ceramic layer or a laser etched ceramic layer 48 may be used to capture the fluid ejected from the ejection head 22. Accordingly, a ceramic layer 48 may be applied to the protective layer 46 and then grit blasted or laser etched to form indentations 50 in the ceramic layer 48 that significantly increase the effective surface area of the heating element 24 as shown. In an alternative embodiment, the protective layer 46 itself may be grit blasted or laser etched as opposed to adding and blasting or etching ceramic layer 48. The grit blasted or laser etched surface of the ceramic layer 48 or the protective layer 46, like the porous layer 44, may be effective to prevent pooling of liquid on the surface thereof and may provide more rapid vaporization of fluid ejected onto the heating element 24.

One method for making a heating element 24, according to an embodiment of the disclosure, is illustrated schematically in FIG. 5-FIG. 7. The heating element 24 includes a ceramic base 40 that contains a high melting point metal heating material such as tungsten, molybdenum, or molybdenum-manganese embedded in a 92% to 96% by weight of alumina ceramic base 40 to form the conductive layer 42. For example, a metal heating resistance slurry of one or more of the foregoing metals may be printed onto a tape casting of a ceramic green body to form the conductive layer 42. Several layers of ceramic green body are then laminated together at a high temperature with the aid of 4% to 8% by weight of a sintering additive to form an alumina ceramic heating substrate of the ceramic base 40. Materials for the ceramic base 40 include, but are not limited to aluminum nitride and cubic boron nitride.

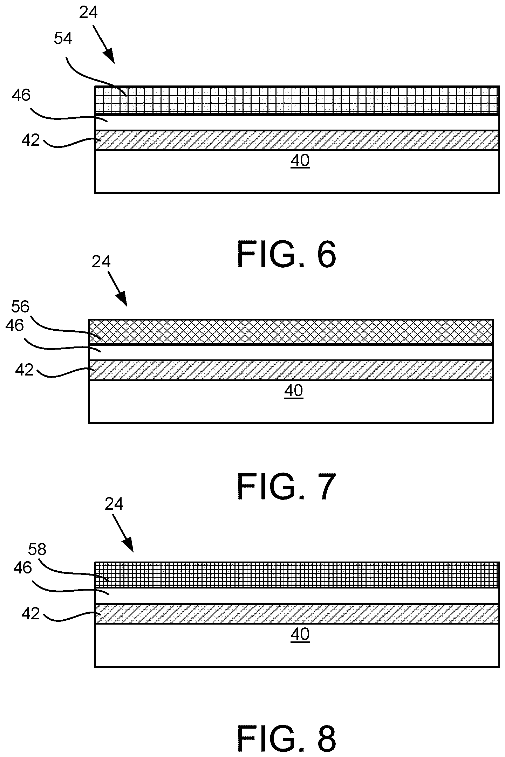

In a next step of the process, as shown in FIG. 6, a layer 54 of glass frit is applied to a protective layer 46 that is applied to the conductive layer 42 to provide the layer 54. The glass frit may be applied as a screen printed paste or slurry to the surface of the heating element 24.

In the final step of the process, as shown in FIG. 7, the glass frit is sintered on the heating element 24 to provide a porous surface 56 having a thickness ranging from about 0.5 millimeters to about 3 millimeters, such as from about 1 millimeter to about 2 millimeters. Upon ejecting fluid from the ejection head 22 onto the heating element 24, the fluid is absorbed by the sintered glass frit layer to form a fluid containing porous layer 58 (FIG. 8). The fluid containing porous layer 58 provides increased effective heating element surface area, so that more efficient evaporation of the liquid may take place.

### DESCRIPTION OF REFERENCE SIGNS

- 10:: vaporization device
- 12:: mouthpiece
- 14:: conduit
- 16:: housing body
- 18:: cartridge cover
- 20:: fluid supply cartridge
- 22:: ejection head/ microfluidic ejection head
- 24:: heating element
- 26:: holder
- 28:: rechargeable power supply
- 30:: main circuit board
- 32:: vaporization driver card
- 34:: damper slide
- 36:: air inlet hole
- 40:: ceramic base
- 42:: conductive layer / resistive or conductive layer
- 44:: fluid absorbing or capturing layer/ porous layer
- 46:: protective layer
- 48:: ceramic layer
- 50:: indentations
- 54:: layer
- 56:: porous surface
- 58:: fluid containing porous layer
- L:: length
- T1:: overall thickness
- T2:: thickness
- W:: width

## Claims

1. A heating element (24) for a vaporization device (10), wherein the heating element (24) comprises:
a conductive layer (42), configured to be disposed on a substrate (40),
wherein the heating element (24) is configured to have an effective surface area for fluid vaporization that is greater than a planar surface area defined by dimensions of the heating element (24);
a protective layer (46), configured to be disposed on the conductive layer (42) and the
protective layer (46) is made of a high temperature material that is substantially non-porous; and a porous layer (44), configured to be disposed on the protective layer (46) and having a porosity of at least about 50%.

2. The heating element (24) of claim 1, wherein
the heating element (24) is configured to have a rectangular shape and has the effective surface area for fluid vaporization defined by an equation ESA > L x W,
wherein ESA is the effective surface area,
L is a length of the heating element (24), and
W is a width of the heating element (24).

3. The heating element (24) of claim 2, wherein
the porous layer (44) has a grit blasted surface texture.

4. The heating element (24) of any one of claim 1 to claim 3, wherein
the porous layer (44) has a thickness (T2) ranging from about 0.5 mm to about 3.0 mm.

5. The heating element (24) of any one of claim 1 to claim 4, wherein
the substrate (40), the conductive layer (42), the protective layer (46) and the porous layer (44) have a combined thickness (T1) ranging from about 4.0 mm to about 1.0 cm.

6. The heating element (24) of any one of claim 1 to claim 5, wherein
the porous layer (44) has a porosity ranging from about 50% to about 95%.

7. The heating element (24) of any one of claim 1 to claim 6, wherein
the conductive layer (42) is a screen printed conductive layer configured to be deposited onto a ceramic substrate.

8. The heating element (24) of any one of claim 1 to claim 7, wherein
the porous layer (44) is a laser etched ceramic layer.

9. The heating element (24) of any one of claim 1 to claim 7, wherein
the porous layer (44) is a coarse grit deposited ceramic layer that is sintered.

10. A vaporization device (10), **characterized in that** the vaporization device (10) comprises:
a housing body (16);
a mouthpiece (12), configured to be attached to the housing body (16); and
a heating element (24) according to any one of claim 1 to claim 9, disposed adjacent to the mouthpiece (12) for vaporizing fluid ejected from an ejection head (22).

11. A method for vaporizing a fluid ejected by an ejection head (22) the method comprises:
providing a vaporization device (10) configured to have the ejection head (22) and a heating element (24) adjacent to the ejection head (22);
ejecting the fluid onto the heating element (24);
activating the heating element (24) during fluid ejection; and
vaporizing substantially all of the fluid using the heating element (24),
wherein the heating element (24) comprises:
a conductive layer (42), configured to be disposed on a substrate (40),
wherein the heating element (24) is configured to have an effective surface area for fluid vaporization that is greater than a planar surface area defined by dimensions of the heating element (24), and wherein the heating element (24) further comprising:
a protective layer (46), configured to be disposed on the conductive layer (42) and the protective layer (46) is made of a a high temperature material that is substantially non-porous; and
a porous layer (44), configured to be disposed on the protective layer (46) and having a porosity of at least about 50%.

12. The method of claim 11, further comprising:
absorbing the fluid by the porous layer (44) of the heating element (24), wherein
the porosity of the heating element (24) is defined by an equation ESA > L x W,
wherein ESA is the effective surface area of the heating element (24),
L is a length of the heating element (24),
W is a width of the heating element (24), and
the heating element (24) is configured to have a rectangular shape and the heating element (24) is exposed to the fluid.

13. The method of claim 11 or claim 12, wherein
the porous layer (44) is deposited as a coarse glass frit that is sintered onto the protective layer (46) of the heating element (24).

## Patentansprüche

1. Ein Heizelement (24) für eine Verdampfungsvorrichtung (10), wobei das Heizelement (24) umfasst:
eine leitende Schicht (42), die so konfiguriert ist, um auf einem Substrat (40) angeordnet zu sein,
wobei das Heizelement (24) so konfiguriert ist, dass es eine effektive Oberfläche zur Fluidverdampfung aufweist, die größer ist als eine planare Oberfläche, die durch die Abmessungen des Heizelements (24) definiert ist;
eine Schutzschicht (46), die so konfiguriert ist, um sie auf der leitenden Schicht (42) anzuordnen , wobei die Schutzschicht (46) aus einem Hochtemperaturmaterial hergestellt ist, das im Wesentlichen nicht porös ist; und
eine poröse Schicht (44), die so konfiguriert ist, um auf der Schutzschicht (46) angeordnet zu sein und eine Porosität von mindestens etwa 50% aufweist.

2. Das Heizelement (24) nach Anspruch 1, wobei
das Heizelement (24) so konfiguriert ist, dass es eine rechteckige Form hat und die effektive Oberfläche für die Fluidverdampfung durch eine Gleichung ESA > L x B definiert ist,
wobei ESA die effektive Oberfläche ist,
L ist eine Länge des Heizelements (24), und
W ist eine Breite des Heizelements (24).

3. Das Heizelement (24) nach Anspruch 2, wobei
die poröse Schicht (44) eine sandgestrahlte Oberflächentextur hat.

4. Das Heizelement (24) nach einem der Ansprüche 1 bis 3, wobei
die poröse Schicht (44) eine Dicke (T2) von etwa 0,5 mm bis etwa 3,0 mm hat.

5. Das Heizelement (24) nach einem der Ansprüche 1 bis 4, wobei
das Substrat (40), die leitende Schicht (42), die Schutzschicht (46) und die poröse Schicht (44) eine kombinierte Dicke (T1) aufweisen, die von etwa 4,0 mm bis etwa 1,0 cm reicht.

6. Das Heizelement (24) nach einem der Ansprüche 1 bis 5, wobei
die poröse Schicht (44) eine Porosität von etwa 50% bis etwa 95% hat.

7. Das Heizelement (24) nach einem der Ansprüche 1 bis 6, wobei
die leitende Schicht (42) eine siebgedruckte leitende Schicht ist, die so konfiguriert ist, um auf ein Keramiksubstrat abgeschieden zu sein.

8. Das Heizelement (24) nach einem der Ansprüche 1 bis 7, wobei
die poröse Schicht (44) eine lasergeätzte Keramikschicht ist.

9. Das Heizelement (24) nach einem der Ansprüche 1 bis 7, wobei
die poröse Schicht (44) eine grobkörnig abgeschiedene Keramikschicht ist, die gesintert ist.

10. Verdampfungsvorrichtung (10), **dadurch gekennzeichnet, dass** die Verdampfungsvorrichtung (10) umfasst:
einen Gehäusekörper (16);
ein Mundstück (12), das so konfiguriert ist, dass es an dem Gehäusekörper (16) befestigt werden kann; und
ein Heizelement (24) nach einem der Ansprüche 1 bis 9, das angrenzend an das Mundstück (12) angeordnet ist, um Fluid zu verdampfen, das aus einem Ausstoßkopf (22) ausgestoßen wird.

11. Verfahren zum Verdampfen eines durch einen Ausstoßkopf (22) ausgestoßenen Fluids, wobei das Verfahren umfaßt:
Bereitstellen einer Verdampfungsvorrichtung (10), die so konfiguriert ist, dass sie den Ausstoßkopf (22) und ein Heizelement (24) neben dem Ausstoßkopf (22) aufweist;
Ausstoßen der Flüssigkeit auf das Heizelement (24);
Aktivieren des Heizelements (24) während des Flüssigkeitsausstoßes; und
Verdampfen von im wesentlichen der gesamten Flüssigkeit unter Verwendung des Heizelements (24),
wobei das Heizelement (24) umfasst:
eine leitende Schicht (42), die so konfiguriert ist, dass sie auf einem Substrat (40) angeordnet werden kann,
wobei das Heizelement (24) so konfiguriert ist, dass es eine effektive Oberfläche zur Fluidverdampfung aufweist, die größer ist als eine planare Oberfläche, die durch die Abmessungen des Heizelements (24) definiert ist, und wobei das Heizelement (24) ferner umfasst:
eine Schutzschicht (46), die so konfiguriert ist, dass sie auf der leitenden Schicht (42) angeordnet werden kann, wobei die Schutzschicht (46) aus einem Hochtemperaturmaterial hergestellt ist, das im Wesentlichen nicht porösist; und
eine poröse Schicht (44), die so konfiguriert ist, dass sie auf der Schutzschicht (46) angeordnet werden kann und eine Porosität von mindestens etwa 50% aufweist.

12. Die Methode von Anspruch 11, weiter umfassend:
Absorbieren des Fluids durch die poröse Schicht (44) des Heizelements (24), wobei
die Porosität des Heizelements (24) ist durch eine Gleichung ESA > L x B definiert,
wobei ESA die effektive Oberfläche des Heizelements (24) ist,
L ist eine Länge des Heizelements (24),
W ist eine Breite des Heizelements (24), und
das Heizelement (24) so konfiguriert ist, dass es eine rechteckige Form hat und das Heizelement (24) dem Fluid ausgesetzt ist.

13. Das Verfahren nach Anspruch 11 oder Anspruch 12, wobei
die poröse Schicht (44) als grobe Glasfritte abgeschieden wird, die auf die Schutzschicht (46) des Heizelements (24) gesintert wird.

## Revendications

1. Un élément chauffant (24) pour un dispositif de vaporisation (10), dans lequel l'élément chauffant (24) comprend:
une couche conductrice (42), configurée pour être disposée sur un substrat (40),
dans lequel l'élément chauffant (24) est configuré pour avoir une surface efficace pour la vaporisation de fluide qui est supérieure à une surface plane définie par les dimensions de l'élément chauffant (24) ;
une couche protectrice (46), configurée pour être disposée sur la couche conductrice (42) et la couche protectrice (46) est faite en matériau haute température qui est substantiellement non poreux ; et
une couche poreuse (44), configurée pour être disposée sur la couche protectrice (46) et ayant une porosité d'au moins environ 50%.

2. L'élément chauffant (24) de la revendication 1, dans lequel
l'élément chauffant (24) est configuré pour avoir une forme rectangulaire et une surface effective pour la vaporisation de fluide définie par une équation ESA> L x W, où ESA est le surface effective,
L est une longueur de l'élément chauffant (24), et
W est une largeur de l'élément chauffant (24).

3. L'élément chauffant (24) de la revendication 2, dans lequel la couche poreuse (44) a une texture de surface grenaillée.

4. L'élément chauffant (24) de l'une quelconque des revendications 1 à 3, dans lequel la couche poreuse (44) a une épaisseur (T2) allant d'environ 0,5 mm à environ 3,0 mm.

5. L'élément chauffant (24) de l'une quelconque des revendications 1 à 4, dans lequel le substrat (40), la couche conductrice (42), la couche protectrice (46) et la couche poreuse (44) ont une épaisseur combinée (T1) allant d'environ 4,0 mm à environ 1,0 cm.

6. L'élément chauffant (24) de l'une quelconque des revendications 1 à 5, dans lequel la couche poreuse (44) a une porosité allant d'environ 50% à environ 95%.

7. L'élément chauffant (24) de l'une quelconque des revendications 1 à 6, dans lequel la couche conductrice (42) est une couche conductrice sérigraphiée configurée pour être déposée sur un substrat en céramique.

8. L'élément chauffant (24) de l'une quelconque des revendications 1 à 7, dans lequel la couche poreuse (44) est une couche de céramique gravée au laser.

9. L'élément chauffant (24) de l'une quelconque des revendications 1 à 7, dans lequel la couche poreuse (44) est une couche de céramique déposée à gros grains qui est frittée.

10. Un dispositif de vaporisation (10), **caractérisé en ce que** le dispositif de vaporisation (10) comprend:
un corps de boîtier (16);
un embout buccal (12), configuré pour être fixé au corps de boîtier (16); et
un élément chauffant (24) selon l'une quelconque des revendications 1 à 10, disposé adjacent à l'embout buccal (12) pour vaporiser le fluide éjecté d'une tête d'éjection (22).

11. Un procédé de vaporisation d'un fluide éjecté par une tête d'éjection (22), le procédé comprenant :
la fourniture d'un dispositif de vaporisation (10) configuré pour avoir la tête d'éjection (22) et un élément chauffant (24) adjacent à la tête d'éjection (22);
éjecter le fluide sur l'élément chauffant (24);
activer l'élément chauffant (24) pendant l'éjection de fluide; et
vaporiser sensiblement tout le fluide à l'aide de l'élément chauffant (24),
dans lequel l'élément chauffant (24) comprenant:
une couche conductrice (42), configurée pour être disposée sur un substrat (40),
dans lequel l'élément chauffant (24) est configuré pour avoir une surface efficace pour la vaporisation de fluide qui est supérieure à une surface plane définie par les dimensions de l'élément chauffant (24), et dans lequel l'élément chauffant (24) comprend en outre :
une couche protectrice (46), configurée pour être disposée sur la couche conductrice (42) et la couche protectrice (46) est faite d'un matériau haute température qui est substantiellement non poreux ; et
une couche poreuse (44), configurée pour être disposée sur la couche protectrice (46) et ayant une porosité d'au moins environ 50%.

12. Le procédé de la revendication 11, comprenant en outre:
l'absorption du fluide par la couche poreuse (44) de l'élément chauffant (24), dans laquelle la porosité de l'élément chauffant (24) est définie par une équation ESA> LxW, dans laquelle ESA est la surface effective de l'élément chauffant (24),
L est une longueur de l'élément chauffant (24),
W est une largeur de l'élément chauffant (24), et
l'élément chauffant (24) est configuré pour avoir une forme rectangulaire forme et l'élément chauffant (24) est exposé au fluide.

13. Le procédé de la revendication 11 ou 12, dans lequel la couche poreuse (44) est déposée sous forme d'une fritte de verre grossière qui est frittée sur la couche protectrice (46) de l'élément chauffant (24).
